# EUROPEAN PATENT APPLICATION

(11) **EP 4 620 464 A1**
(43) Date of publication of application: **24.09.2025**
(21) Application number: 24855540.1
(22) Date of filing: 20.07.2024
(51) Int. Cl.: A61K 31/155, A61K 9/20, A61K 9/28, A61K 9/48, A61P 3/00, A61P 3/10

(54) **METFORMIN HYDROCHLORIDE ENTERIC-COATED PREPARATION**

(30) Priority: 22.08.2023 CN 202311062476
(71) Applicant: Yayan (Tianjin) Biomedical Technology Center (Limited Partnership), Tianjin 301721 (CN)
(72) Inventor: GAO, Qian, Tianjin 301721 (CN); WANG, Wei, Tianjin 301721 (CN); DONG, Tao, Tianjin 301721 (CN)
(74) Representative: Danubia Patent & Law Office LLC
(86) International application number: PCT/CN2024/106616
(87) International publication number: WO 2025/039813

(57) **Abstract**

The invention provides a new enteric-coated preparation of metformin hydrochloride, which is an oral dosage form made of a therapeutically effective amount of a biguanide compound and a pharmaceutically acceptable excipient, characterized in that the said enteric-coated preparation of metformin hydrochloride has a dissolution rate of no more than 15% in a pH 4.5 medium within 120 minutes, and a dissolution rate of no less than 85% in a pH 6.8 medium within 20 minutes, preferably a dissolution rate of no more than 7.5% in a pH 4.5 medium within 120 minutes, and a dissolution rate of no less than 85% in a pH 6.8 medium within 15 minutes. The enteric-coated preparation of metformin hydrochloride with the drug dissolution technical requirements of the present invention improves the gastrointestinal tolerance of metformin hydrochloride without reducing or even improving the hypoglycemic efficacy, reduces adverse reactions caused by the drug, and can be taken before, after or during meals, thereby improving the compliance of patients with medication.

## Description

### TECHNICAL FIELD

The invention provides an enteric-coated preparation of a biguanide compound, in particular provides an enteric-coated preparation of metformin hydrochloride, belonging to the field of pharmaceutical preparations.

### BACKGROUND OF THE INVENTION

Metformin is an oral biguanide hypoglycemic drug. Its hydrochloride, namely metformin hydrochloride, is commonly used clinically. Early studies attributed metformin's hypoglycemic effect primarily to systemic effect produced after drug absorption, such as inhibiting gluconeogenesis, reducing hepatic glucose output, acting on peripheral tissues (muscle, adipose tissue), promoting glucose utilization and improving insulin sensitivity. Recent studies have found that the intestinal mediated mechanism produced by unabsorbed drugs (such as increasing GLP-1 and PYY levels, affecting intestinal flora and inhibiting intestinal wall cells from taking up glucose) may be more important.

In the diabetes diagnosis and treatment guidelines formulated by many countries and international organizations, metformin hydrochloride is recommended as the first - line drug for controlling hyperglycemia in patients with type II diabetes and as the basic drug in combination therapy. Glucophage (metformin hydrochloride tablets), the originator product of metformin, has accumulated extensive clinical experience over decades and is supported by robust evidence-based medical data, is the standard drug for type II diabetes globally and the most commonly used control drug in clinical trials.

However, some adverse reactions of metformin restrict its use. Some patients cannot tolerate these adverse reactions and have to stop taking the drug. The main adverse reactions of metformin include gastrointestinal reactions, such as diarrhea, nausea, vomiting, abdominal distension, abdominal pain, indigestion, abdominal discomfort, etc., and nervous system adverse reactions, such as dizziness and headache. In addition, it can also cause flu-like symptoms and vitamin B₁₂ absorption disorders, and may also cause some rare but more serious adverse reactions, such as lactic acidosis. Gastrointestinal adverse reactions (such as nausea, abdominal pain, abdominal discomfort, etc.) are the most common adverse reactions of metformin, with an incidence of 20-30%, and are also the most common cause of patient intolerance.

In order to reduce the adverse reactions caused by the use of metformin, many studies on its enteric-coated preparations have been performed in the prior art. Enteric-coated preparations refer to preparations that do not release or almost do not release the drug substance in the stomach within a specified time, but can release most or all in a certain part of the intestine after entering the intestine.

Early studies and reports on enteric-coated metformin hydrochloride preparations, such as CN101190179B, only focused on whether the drug was not released or almost not released in 0.1 mol/L hydrochloric acid medium and whether it was completely released in pH 6.8 phosphate buffer. Subsequent studies and reports, such as CN101785763B, disclosed enteric-coated sustained-release tablets of metformin hydrochloride, which released about 30% of the drug in 1 hour, about 50% in 3 hours, and more than 85% in 10 hours in pH 6.8 phosphate buffer, claiming that the sustained release of the drug improved bioavailability and avoided the possible side effects caused by excessive plasma concentration in a short period of time. CN102357088A disclosed an enteric-coated metformin hydrochloride preparation that was rapidly released in pH 5.5-6.8 buffers and the release was more than 80% even in a pH 5.5 phosphate buffer after 45 minutes, claiming that the drug could avoid adverse reactions and the rapid release characteristics can ensure effective release in intestinal fluid with a pH value of 5.5-6.8 and can more effectively ensure clinical efficacy of the product. CN106420653A discloses metformin hydrochloride enteric-coated tablets with a dissolution amount of 20-30% in pH 6.8 phosphate buffer in 1 hour, 45-55% in 2 hours and more than 85% in 4 hours, claiming to have low adverse reactions and be suitable for patients with contraindications to metformin. CN111888339A discloses metformin hydrochloride enteric-coated tablets with fast release which release more than 85% in pH 4.5-5.0 buffer in 15-30 minutes, claiming to have improved bioavailability and be bioequivalent to the original drug. The enteric-coated preparations of metformin hydrochloride disclosed in these prior arts meet the common requirements that enteric-coated preparations do not release or almost release no drugs in acidic medium of pH 1.0-3.0, and release most or all of the drugs in buffered salt medium of pH 6.8. However, the relationship between the release characters (such as the speed or degree of release in the medium, etc.) and the therapeutic effects achieved by the enteric-coated preparations is contradictory reported in the prior arts, in which some have rapid release characters ensure efficacy and /or safety, while others have slower release characters ensure efficacy and /or safety, which are obviously contradictory to each other and lack further verification.

In addition, ELCELYX THERAPEUTICS, INC. disclosed an enteric-coated preparation of metformin hydrochloride in WO2013103384A, WO2013103919A and WO2014107617A. The preparation is a delayed release preparation (DR) designed to push the drug into the distal small intestine to release the active drug, thereby improving the gastrointestinal tolerance of the biguanide compound and reducing the adverse events caused by the biguanide compound. WO2013103384A and WO2013103919A disclose a delayed-release formulation having a relative bioavailability reduced by 20% to 60%, preferably 40% to 60%, compared to a conventional formulation, i.e., an immediate-release formulation (IR), having an equal amount of the biguanide compound. WO2014107617A discloses an enteric-coated oral dosage form containing a biguanide compound, wherein the dosage form showed a lag phase in drug release of at least about 5 or 10 minutes after contact with a pH of 6.0, 6.5, 6.8 or 7.0, preferably a lag phase of at least about 15 or 20 minutes after contact with the desired pH, and more preferably a lag phase of at least about 25 or 30 minutes after contact with a pH of 6.0, 6.5, 6.8 or 7.0; in a preferred Example, the enteric-coated oral dosage form containing a biguanide compound release less than 15% of the drug after two hours at acid pH and a lag phase of at least ten or fifteen minutes at pH 6.8, and at least 60% of the biguanide compound is released after the lag phase and within 60 minutes at pH 6.8, and at least 90% of the biguanide compound is released within 90 to 120 minutes at pH 6.8. In another preferred Example, the enteric-coated oral dosage form comprising a biguanide compound releases less than 15 %, 10%, or 5% of the biguanide compound in two hours at acidic pH, 30 minutes at pH 5.5, and a lag period of at least ten or fifteen minutes at pH 6.8, releases at least 60 % of the biguanide compound after the lag period and within 60 minutes at pH 6.8, and releases at least 90 % of the biguanide compound within 90 to 120 minutes at pH 6.8.

The prior art such as WO2014107617A discloses enteric-coated preparations of metformin hydrochloride (delayed-release preparations, DR), which claim that the hypoglycemic efficacy is not less than that of the same dose of ordinary metformin hydrochloride tablets, namely, immediate-release preparations (IR), and even has an increased efficacy. Moreover, compared with IR preparations, DR preparations of metformin hydrochloride reduce the relative bioavailability and systemic exposure of the drug, improve the gastrointestinal tolerance and reduce adverse drug events. However, the hypoglycemic efficacy of these studies is evaluated by fasting plasma glucose (FPG) or GLP-1, PYY, etc., which has poor accuracy, especially when the number of cases is small. Therefore, the hypoglycemic efficacy of DR preparations of metformin hydrochloride described is not confirmed clinically. For example, Elcelyx later conducted a 16-week clinical study on metformin hydrochloride DR preparations with an expanded number of cases (see Robert R. Henry et al., Improved glycemic control with minimal systemic metformin exposure: Effects of Metformin Delayed-Release (Metformin DR) targeting the lower bowel over 16 weeks in a randomized trial in subjects with II-type diabetes, PLOS ONE, published on September 25, 2018, https://doi.org/10.1371/journal.pone.0203946), and used the clinical gold standard glycated hemoglobin (HbA1c) as the main efficacy indicators, the results showed that the metformin hydrochloride DR preparation (prepared by the technical solution disclosed in the patent document) failed to show the expected clinical effect. In the 16-week clinical trial, the best dosing regimen, i.e. giving patients metformin hydrochloride DR every morning, the hypoglycemic efficacy of DR 1500 mg was much lower than that of the control group (metformin hydrochloride IR 2000 mg, administration according to the labeling instructions, 1000 mg in the morning and 1000 mg in the evening), and the reduction in efficacy was greater than the reduction in dosage.

### SUMMARY OF THE INVENTION

The present invention aims to provide a new enteric-coated preparation of biguanide compound, and in particular an enteric-coated preparation of metformin hydrochloride. Compared with metformin hydrochloride immediate-release preparation (IR) or enteric-coated preparations (DR) with different release rates, in the present invention the gastrointestinal tolerance of metformin hydrochloride is improved without reducing or even improving the hypoglycemic efficacy, and the adverse reactions caused by the drug are reduced, which is significantly different from enteric-coated preparations without the release characteristics said in the present invention.

The technical solution of the present invention is as follows:
The invention provides a biguanide compound enteric-coated preparation, which is an oral dosage form made of a therapeutically effective amount of a biguanide compound and a pharmaceutically acceptable excipient, characterized in that the dissolution rate of the biguanide compound in the enteric-coated preparation in a pH 4.5 medium within 120 minutes is not higher than 15%, and the dissolution rate of the biguanide compound in a pH 6.8 medium within 20 minutes is not lower than 85%. More preferably, the dissolution rate of the biguanide compound in the said enteric-coated preparation in a pH 4.5 medium within 120 minutes is not higher than 7.5%, and the dissolution rate of the biguanide compound in a pH 6.8 medium within 15 minutes is not less than 85%. Preferably, the biguanide compound is metformin or a pharmaceutically acceptable salt thereof. More preferably, the biguanide compound is metformin hydrochloride.

In the present invention, "dissolution rate" is also referred to as "release rate", and "dissolution" is also referred to as "release", which have the same meaning in terms of expression. The dissolution or release of a drug can be determined according to the commonly used determination methods in the art, for example, according to the dissolution and release determination method of the first method of General Chapter 0931 of the Chinese Pharmacopoeia (2020 Edition).

In the present invention, the pH 4.5 medium is preferably a pH 4.5 acetate buffer, and the pH 6.0 medium or the pH 6.8 medium is preferably a pH 6.0 phosphate buffer or a pH 6.8 phosphate buffer.

The enteric-coated preparation of the present invention is a preparation having the drug release characteristics of the present invention while meeting the general requirements for drug release of enteric-coated preparations. The general requirements for drug release of enteric-coated preparations refer to a preparation that does not release or hardly releases the drug in a specified acidic medium (e.g., pH 1.0-3.0), but releases most or all the drug in a near-neutral medium (e.g., pH 6.0-6.8 phosphate buffer) within a required time.

The enteric-coated preparation of the present invention described above may be in the form of tablets, capsules (including enteric-coated capsules or micro-pellet capsules) or granules.

Preferably, the enteric-coated preparation of the present invention has a relative bioavailability of 60% to 80% compared with an immediate-release preparation (or a conventional preparation, such as Glucophage) having an equal amount of the biguanide compound.

As another object of the present invention, there is also provided the use of a biguanide compound or a salt thereof in the preparation of a medicament for reducing the risk of adverse events caused by administering the biguanide compound to an individual in need thereof, wherein the medicament is the enteric-coated preparation described above in the present invention.

As another object of the present invention, there is also provided use of a biguanide compound or a salt thereof in the preparation of a medicament for treating metabolic disorders, wherein the medicament is the enteric-coated preparation described above in the present invention.

As another object of the present invention, there is also provided use of a biguanide compound or a salt thereof in the preparation of a medicament for diabetes, wherein the medicament is the enteric-coated preparation described above in the present invention.

As another object of the present invention, there is also provided use of a biguanide compound or a salt thereof in the preparation of a medicament for reducing body weight, wherein the medicament is the enteric-coated preparation described above in the present invention.

As another object of the present invention, there is also provided the use of a biguanide compound or a salt thereof in preparing a drug that can be taken before, after or during meals to improve the patient's compliance with medication, wherein the drug is the enteric-coated preparation described above in the present invention.

The enteric-coated preparation described above in the present invention is an oral dosage form made of a therapeutically effective amount of a biguanide compound and a pharmaceutically acceptable excipient. The dosage or therapeutically effective amount of a biguanide compound (e.g., metformin hydrochloride) in a unit preparation, its therapeutic use and its adverse reactions to medication are prior art known to those skilled in the art. The prior art mentioned in the specification and its relevant contents disclosed will be introduced into this application by reference. Excipients used in the enteric-coated preparation of the present invention, such as fillers and disintegrants used in tablets, gelatin used in capsules, flavoring agents used in granules, and related enteric materials, etc., are either commercially supplied in the market, or prepared or formulated according to prior art. Those skilled in the art can prepare them according to the general preparation process of the enteric-coated preparation in the art. For enteric-coated tablets with low weight gain in coating, since it is often not easy to meet the acid resistance requirements or the dissolution requirements of pH 4.5, it may be necessary to control appropriate process parameters. For example, the liquid supply speed of the peristaltic pump should be checked before coating to ensure stable and accurate liquid supply, and the atomization pressure and fan pressure are adjusted to make the coating liquid have a better atomization state. In the early stage of coating, the liquid supply volume can be increased, and the tablet bed temperature can be controlled at the upper limit so that the coated tablets are quickly covered with a layer of coating material. During the entire coating process, the air intake humidity is strictly controlled to ensure the density of the coating film, or an isolation coat is added to obtain a metformin hydrochloride enteric-coated preparation that meets the requirements of the present invention.

The present invention has surprisingly found that the enteric-coated metformin hydrochloride preparation having the specific drug release technical requirements of the present invention can produce the best therapeutic effect and the lowest adverse reaction. The said specific drug release technical requirements for the enteric-coated preparation of the present invention are: the dissolution rate of metformin hydrochloride in a pH 4.5 medium within 120 minutes is not higher than 15%, and the dissolution rate of metformin hydrochloride in a pH 6.8 medium within 20 minutes is not less than 85%, more preferably, the dissolution rate of the biguanide compound in a pH 4.5 medium within 120 minutes is not higher than 7.5%, and the dissolution rate of metformin hydrochloride in a pH 6.8 medium within 15 minutes is more than 85%. Clinical trials, pharmacokinetic studies, and in vitro and in vivo correlation studies have shown that the enteric-coated metformin hydrochloride preparation having the above-mentioned technical requirements for drug release of the present invention, compared with the metformin hydrochloride IR preparation (e.g., Glucophage) or other enteric-coated preparations with different release rates, can alleviate or reduce the adverse reactions caused by metformin hydrochloride, especially gastrointestinal adverse reactions, without reducing or even improving the hypoglycemic efficacy. Moreover, the enteric-coated preparation can be taken before, after, or during meals, thereby improving the patient's compliance with medication, which is significantly different from enteric-coated preparations without the above-mentioned release characteristics.

In particular, the present invention has shown through in vitro and in vivo correlation studies that dissolution in a pH 6.8 medium is highly correlated with drug absorption. Bioavailability (BA) studies have shown that the enteric-coated metformin hydrochloride preparations with different compositions that meet the above-mentioned drug release technical requirements of the present invention are all bioequivalent to the preparation of the preferred embodiment (E1). In the pharmaceutical field, drug dissolution or release behavior is often used to distinguish the difference of preparations. The difference in preparation composition and/or production process is also distinguished by drug dissolution or release behavior. It is expected that the drug dissolution or release behavior can be used to predict the in vivo absorption or bioavailability (BA) of the drug. Compared with BA, testing of drug dissolution or release is much simpler and faster. If the correlation between drug dissolution and BA can be established, it is of great value for determining the appropriate composition and process, predicting drugability, etc. The correlation between in vitro dissolution and in vivo absorption of the enteric-coated metformin hydrochloride preparation has been established in the present invention through a lot of studies. The present invention provides a method for predicting absorption, clinical efficacy and safety in human by in vitro dissolution for the enteric-coated metformin hydrochloride preparation, demonstrating the clinical advantages of the enteric-coated metformin hydrochloride preparation with the above-mentioned drug release technical requirements of the present invention.

### BRIEF DESCRIPTION OF THE DRAWINGS

Figure 1: HbA1c reduction during 12 weeks of treatment with E1, D2, D5 and Glucophage (R)
Figure 2: FPG change of E1, D2, D5 and Glucophage (R)
Figure 3: AE of E1, D2, D5 and Glucophage (R)
Figure 4: Dissolution curves (pH 6.8) of E1, D2, and D5
Figure 5: Plasma concentration - time curves of E1, D2, D5 and Glucophage (R)
Figure 6: Dissolution curves (pH 6.8) of E1, E10, E13 and D4
Figure 7: Plasma concentration - time curves of E1, E10, E13 and D4
Figure 8: Dissolution curves (pH 6.8) of E1, E4 and D1
Figure 9: Plasma concentration - time curves of E1, E4 and D1
Figure 10: Dissolution curves (pH 6.8) of E1 and E22
Figure 11: Plasma concentration - time curves of E1 and E22
Figure 12: Distribution and flow chart of subjects

### DETAILED DESCRIPTION OF THE EXAMPLES

The following specific examples are only used to explain or illustrate the content of the present invention. Obviously, the implementation mode of the present invention is not limited to the specific examples listed, so they should not be understood as constituting any limitation on the protection scope of the claims of this patent application.

### Example 1 (E1): Metformin hydrochloride enteric-coated tablets

### Composition of tablet core:

| **Ingredients** | **Dosage (mg)** |
|---|---|
| Metformin Hydrochloride | 500.00 |
| Corn starch | 16.54 |
| Mannitol | 20.00 |
| Sodium Starch Glycolate | 16.68 |
| Magnesium Stearate (Added externally) | 2.78 |
| Purified water | Moderate |
| Tablet weight | 556 |

Preparation method: Metformin hydrochloride is crushed and passed through an 80 -mesh sieve. The crushed API and the internally added excipients are placed in a wet granulator for mixing for 5 minutes, and then purified water is added for wet granulation. The wet granules are granulated using a granulator (with a 5×5mm square hole screen installed) and then put into a fluid bed drier for drying. The moisture content of the dry granules is controlled within the range of 1.0 to 4.0%. After the dry granules are granulated (the granulator is equipped with a 1.5mm round hole screen), magnesium stearate is added and mixed in a blender for 5 minutes. A 12mm round punch is used for tableting, and the hardness of the plain tablets is controlled at 8 to 11 kg.

According to the disintegration time test method in General Chapter 0921 of Part IV of the 2020 edition of the "Chinese Pharmacopoeia", it was measured that the plain tablets disintegrated completely within 2 to 4 minutes.

### Composition of enteric coating solution:

| **Ingredients** | **Dosage (g)** |
|---|---|
| Methacrylic acid ethyl acrylate copolymer (L100-55) | 6.00 |
| Triethyl citrate | 0.60 |
| Talc | 1.50 |
| Ethanol (90%) | 91.90 |

Enteric layer coating: Add methacrylic acid ethyl acrylate copolymer (L100-55) to ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 4.5% (7.2mg/cm²).

### Example 2 (E4): Metformin hydrochloride enteric-coated tablets

The total mixed granules were obtained according to the core composition and preparation method of Example 1. The hardness of the plain tablets was controlled at 15-18 kg during tableting (a small amount of binder was added if necessary). According to the disintegration time test method in General Chapter 0921 of Part IV of the 2020 edition of the "Chinese Pharmacopoeia", it was measured that the plain tablets disintegrated completely within 6-8 minutes. The enteric coating solution composition, process and weight gain in coating were the same as those in Example 1.

### Example 3 (E10): Metformin hydrochloride enteric-coated tablets

The tablet core was prepared according to the tablet core composition and preparation method of Example 1.

### Composition of enteric coating solution:

| **Ingredients** | **Dosage ( g )** |
|---|---|
| Hydroxypropyl methylcellulose phthalate ( HP-50 ) | 8.00 |
| Triethyl citrate | 0.40 |
| Talc | 2.40 |
| Ethanol (80%) | 89.20 |

Enteric layer coating: Add Hydroxypropyl Methylcellulose Phthalate to ethanol(80%), stir until completely dissolved, then add triethyl citrate and talc in turn and stir until evenly dispersed. Use a coating machine to coat the enteric layer, dry after coating to remove residual solvent, and the weight gain of the enteric layer coating is 8.0 % (12.8 mg/cm²).

### Example 4 (E12): Metformin hydrochloride enteric-coated tablets

The tablet core was prepared according to the tablet core composition and preparation method of Example 1, and the enteric coating was prepared according to the composition of enteric coating solution and process of Example 3, and the weight gain in coating was 4.0 % (6.4 mg/cm²).

### Example 5 (E13): Metformin hydrochloride enteric-coated tablets

The tablet core was prepared according to the tablet core composition and preparation method of Example 1.

### Composition of enteric coating solution:

| **Ingredients** | **Dosage ( g )** |
|---|---|
| Methacrylic acid methyl methacrylate copolymer (Eudragit L100 ) | 6.00 |
| Triethyl citrate | 0.90 |
| Talc | 1.50 |
| Ethanol (90%) | 91.60 |

Enteric layer coating: Add methacrylic acid methyl methacrylate copolymer into ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 3.5% (5.6 mg/cm²).

### Example 6 (E15): Metformin hydrochloride enteric-coated tablets

The tablet core was prepared according to the tablet core composition and preparation method of Example 1.

### Composition of isolation layer coating solution:

| **Ingredients** | **Dosage ( g )** |
|---|---|
| Gastric soluble film coating premix (Opadry 85F ) | 15.0 |
| Purified water | 85.0 |

Isolation layer coating: Add the gastric soluble film coating premix into purified water, stir to disperse evenly, and sieve through 80 mesh before coating. Use a coating machine to coat the isolation layer, dry it after coating to remove the residual moisture, and the weight gain in coating is 2.0%.

### Composition of enteric coating solution:

| **Ingredients** | **Dosage ( g )** |
|---|---|
| Methacrylic acid methyl methacrylate copolymer (Eudragit L100 ) | 6.00 |
| Triethyl citrate | 0.6 |
| Talc | 1.50 |
| Ethanol (90%) | 91.90 |

Enteric layer coating: Add methacrylic acid methyl methacrylate copolymer into ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 2.5% (4.0 mg/cm²).

### Example 7 (E22) : Metformin hydrochloride enteric-coated (pellets) capsules

### Composition:

| **Ingredients** | | **Dosage (mg )** |
|---|---|---|
| Pill core | Sucrose pellet core (600~710µm) | 60.00 |
| Drug-loading layer | Metformin Hydrochloride | 250.00 |
| | Hydroxypropyl methylcellulose E5 | 12.50 |
| | Cross-linked polyvinylpyrrolidone XL-10 | 12.50 |
| | Purified water | 725.00 |
| | Total (solid matter) | 335.00 |
| Enteric layer | Hydroxypropyl methylcellulose phthalate (HP-55) | 62.04 |
| | Triethyl citrate | 3.10 |
| | Talc | 18.61 |
| | Ethanol (80%) | 691.71 |
| | Total (solid matter) | 418.75 |

### Preparation method:

### 1. Drug-carrying layer coating

(1) Preparation of coating solution: Dissolve hydroxypropyl methylcellulose and metformin hydrochloride in purified water, add cross-linked polyvinylpyrrolidone and stir to make it dispersed evenly, and pass the coating solution through an 80-mesh sieve for later use.
(2) Coating: Place the sucrose pellet core in a fluidized bed coating machine, adjust the air volume and atomization pressure to keep the pellets in a good fluidized state, set the air temperature and peristaltic pump speed, and coat. The weight gain in coating is about 458.3%. After coating, stop spraying and dry, and screen out qualified pellets.

### 2. Enteric layer coating

(1) Preparation of coating solution: Add hydroxypropyl methylcellulose phthalate to ethanol (80%), stir until completely dissolved, then add triethyl citrate and talc in sequence and stir until evenly dispersed.
(2) Coating: Place qualified drug-loaded pills in a fluidized bed coating machine, adjust the air volume and atomization pressure to keep the pellets in a good fluidized state, set the air temperature and peristaltic pump speed for coating. The weight gain in coating is about 25.0%. After coating, stop spraying and dry, and screen out qualified pellets.

### 3. Capsule filling: Use No. 0 gelatin capsules for filling.

### Comparative Example 1 (D1): Metformin hydrochloride enteric-coated tablets

The composition of the tablet core is based on Glucophage (Metformin Hydrochloride Tablets), specifically:

| **Ingredients** | **Dosage (mg )** |
|---|---|
| Metformin Hydrochloride | 500.00 |
| Povidone K30 | 20.00 |
| Magnesium Stearate (Added externally) | 4.00 |
| Purified water | Moderate |
| Tablet weight | 524 |

Preparation method: dissolve povidone K30 in purified water to prepare a binder for use, grind metformin hydrochloride and pass it through a 100 -mesh sieve, place the ground raw materials in a fluid bed granulating drier, and then spray the binder for one-step granulation, control the moisture content of the dry granules within the range of 1.0 to 4.0%, granulate the dry granules (the granulator is equipped with a 1.5 mm round hole screen), add magnesium stearate, mix in a blender for 5 minutes, and then use a 12 mm round punch for tableting, and the hardness of the plain tablets is controlled within the range of 11 to 15 kg.

According to the disintegration time test method in General Chapter 0921 of Part IV of the 2020 edition of the "Chinese Pharmacopoeia", it was measured that the plain tablets disintegrated completely within 12 to 15 minutes.

The composition of the enteric layer coating solution, process and weight gain in coating are the same as those in Example 1.

### Comparative Example 2 (D2): Metformin Hydrochloride Enteric-coated Tablets

Tablet cores were prepared according to the tablet core composition and preparation method of Example 1, and enteric coating was performed according to the composition of the enteric layer coating solution and process of Example 10, with the weight gain in coating being 2.5% (4.0 mg/cm² ).

### Comparative Example 3 (D4): Metformin Hydrochloride Enteric-coated Tablets

Tablet cores were prepared according to the tablet core composition and preparation method of Example 1.

### Composition of enteric coating solution:

| **Ingredients** | **Dosage ( g )** |
|---|---|
| Methacrylic acid methyl methacrylate copolymer (L100 ) | 6.00 |
| Triethyl citrate | 0.6 |
| Talc | 1.50 |
| Ethanol (90%) | 91.90 |

Enteric layer coating: Add methacrylic acid methyl methacrylate copolymer into ethanol(90%) and stir to completely dissolve. Then add triethyl citrate and talc in turn and stir to disperse evenly. Use a coating machine to coat the enteric layer. After coating, dry to remove residual solvent. The weight gain of the enteric layer coating is 6.5 % (10.4 mg/cm²).

### Comparative Example 4 (D5): Metformin Hydrochloride Enteric-coated Tablets

The tablet core was prepared according to the tablet core composition and preparation method of Comparative Example 1.

### Composition of isolation layer coating solution:

| **Ingredients** | **Dosage ( g )** |
|---|---|
| Gastric soluble film coating premix (Opadry 85F ) | 15.0 |
| Purified water | 85.0 |

Separation layer coating: Add the gastric soluble film coating premix into purified water, stir to disperse evenly, and sieve through 80-mesh before coating. Use a coating machine to coat the isolation layer, dry it after coating to remove the residual moisture, and the weight gain in coating is 1.1%.

### Composition of enteric coating solution:

| **Ingredients** | **Dosage ( g )** |
|---|---|
| Methacrylic acid, methyl acrylate and methyl methacrylate copolymer (FS30D ) | 13.33 |
| Methacrylic acid ethyl acrylate copolymer (L30D-55 aqueous dispersion) | 20.00 |
| Triethyl citrate | 1.0 |
| Talc | 2.50 |
| Purified water | 63.17 |

Enteric layer coating: Add triethyl citrate and talc to purified water, use a high shear homogenizer to fully homogenize for 5 to 10 minutes, then slowly pour the above liquid into the Eudragit FS30D and L30D-55 aqueous dispersion, slowly stir to disperse evenly, and pass through an 80- mesh sieve before coating. Use a coating machine to coat the enteric layer, dry it after coating to age the enteric layer, and the enteric layer weight gain in coating is 3.8% (6.1mg/cm²).

### Methods of dissolution test and results

Refer to the dissolution test conditions of metformin hydrochloride enteric-coated tablets in the 2020 edition of the "Chinese Pharmacopoeia" and determine it according to the dissolution and release determination method (General Chapter 0931 Method 1).

### Dissolution in acid:

Dissolution conditions: Use 900 ml of 0.1 mol/L hydrochloric acid solution as the dissolution medium, rotate at 100 rpm (revolutions per minute), follow the prescribed procedures, and take samples after 2 hours.

Test solution: Take 20 ml of the dissolution, filter, discard 10 ml of the initial filtrate, and take the subsequent filtrate.

Reference solution: Take an appropriate amount of metformin hydrochloride reference substance, weigh it accurately, dissolve it in 0.1 mol/L hydrochloric acid solution and quantitatively dilute it to make a solution containing about 28 µg per 1 ml.

Determination method: Take the test solution and the reference solution, measure the absorbance at a wavelength of 233nm respectively, and calculate the dissolution amount.

Limit: Not more than 10% of the labeled amount.

**Dissolution in pH 4.5 buffer:** Use 900 ml of pH 4.5 acetate buffer as the dissolution medium, *Basket - rotating method,* rotation speed of 100 rpm, follow the prescribed procedures, and take samples after 2 hours. The test solution, the reference solution and the determination method are the same as determined in the pH 6.0 and pH 6.8 medium.

**Dissolution in pH 6.0 and pH 6.8 buffers:** Take the rotating basket after 2 hours under the acid-resistant testing, and immediately immerse it in 900ml of buffer preheated to 37°C±0.5°C. Keep the speed unchanged and continue to follow the prescribed procedures. Sampling is carried out after 5, 10, 15, 20, 30, 45 and 60 minutes.

Test solution: Take 20 ml of the dissolution, filter, discard 10 ml of the primary filtrate, accurately measure an appropriate amount of the subsequent filtrate, and quantitatively dilute with water to make a solution containing approximately 5 µg of metformin hydrochloride per 1 ml.

Reference solution: Take an appropriate amount of metformin hydrochloride reference substance, weigh accurately, dissolve in water and quantitatively dilute to make a solution containing about 5µg per 1ml.

Determination method: Take the test solution and the reference solution, measure the absorbance at a wavelength of 233nm respectively, and calculate the dissolution amount.

### Preparation of dissolution medium:

pH 4.5 medium (acetate buffer): weigh 1.80g of anhydrous sodium acetate (or 2.99g of sodium acetate trihydrate), add 1.596ml of glacial acetic acid to 1L of degassed purified water, stir until completely dissolved, and adjust the pH value to 4.50 with glacial acetic acid.

pH 6.0 medium (phosphate buffer): Take 6.80g of potassium dihydrogen phosphate and 0.224g of sodium hydroxide and dissolve them in 1000ml of purified water. If necessary, adjust the pH value to 6.0 ± 0.05 with 2mol/L hydrochloric acid solution or 2mol /L sodium hydroxide solution.

pH 6.8 medium (phosphate buffer): Take 0.1mol/L hydrochloric acid solution and 0.2mol/L sodium phosphate solution, mix them evenly at a ratio of 3 : 1, and adjust the pH value to 6.8±0.05 with 2mol/L hydrochloric acid solution or 2mol/L sodium hydroxide solution if necessary.

The dissolution test results of the metformin hydrochloride enteric-coated tablets of the examples and the comparative examples: Results of the acid resistance test were suitable. The dissolution results in the acid-resistant and pH 4.5 medium are shown in Table 1 (the value of the acid-resistant is the average value of the acid-resistant data in the pH 6.0 and pH 6.8 medium). The dissolution results in the pH 6.0 and pH 6.8 medium are shown in Table 2 and Table 3 (the dissolution at 0 in the chart is the acid-resistant 2h test value; 6 preparation units are used for each dissolution curve measurement ).

**Table 1: Dissolution of the examples and comparative examples in pH 1.0 and pH 4.5 medium for 2 hours (%)**

| | Example 1 (E1) | Example 2 (E4) | Example 3 (E10) | Example 4 (E12) | Example 5 (E13) | Example 6 (E15) | Example 7 (E22) | Comparative Example 1 (D1) | Comparative Example 2 (D2) | Comparative Example 3 (D4) | Comparative Example 4 (D5) |
|---|---|---|---|---|---|---|---|---|---|---|---|
| pH1.0 | 0.3 | 0.2 | 0.0 | 2.3 | 2.7 | 2.9 | 2.2 | 0.5 | 6.3 | 0.0 | 0.0 |
| pH4.5 | 3.9 | 2.9 | 0.8 | 11.9 | 6.9 | 11.2 | 6.3 | 1.8 | 21.6 | 0.0 | 1.5 |

**Table 2: Dissolution of Examples and Comparative Examples in pH 6.0 Medium (%)**

| Time(min) | **0** | **5** | **10** | **15** | **20** | **30** | **45** | **60** |
|---|---|---|---|---|---|---|---|---|
| Example 1(E1) | 0.5 | 0.5 | 1.2 | 9.3 | 35.0 | 93.5 | 97.1 | 98.6 |
| Example 2(E4) | 0.3 | 0.6 | 1.1 | 8.3 | 22.2 | 65.8 | 95.9 | 99.5 |
| Example 3(E10) | 0.0 | 0.1 | 2.5 | 38.1 | 85.5 | 96.0 | 100.2 | 101.2 |
| Example 4(E12) | 2.2 | 8.7 | 72.8 | 91.1 | 96.2 | 98.2 | 98.1 | 97.4 |
| Example 5(E13) | 2.5 | 0.1 | 0.6 | 1.0 | 2.3 | 5.0 | 13.5 | 32.7 |
| Example 6(E15) | 4.7 | 0.1 | 0.1 | 0.2 | 0.3 | 3.6 | 13.8 | 41.7 |
| Example 7(E22) | 2.3 | 1.5 | 3.7 | 12.7 | 28.9 | 86.8 | 95.8 | 98.7 |
| Comparative Example 1(D1) | 0.4 | 0.5 | 0.9 | 2.6 | 11.3 | 38.5 | 79.3 | 95.8 |
| Comparative Example 2(D2) | 7.2 | 16.2 | 79.3 | 93.1 | 94.3 | 93.9 | 94.0 | 94.1 |
| Comparative Example 3(D4) | 0.0 | 0.1 | 0.0 | 0.0 | 0.1 | 0.2 | 1.8 | 3.1 |
| Comparative Example 4(D5) | 0.0 | 0.0 | 0.1 | 0.2 | 0.2 | 0.3 | 0.4 | 0.5 |

**Table 3: Dissolution of Examples and Comparative Examples in pH 6.8 Medium (%)**

| Time(min) | **0** | **5** | **10** | **15** | **20** | **30** | **45** | **60** |
|---|---|---|---|---|---|---|---|---|
| Example 1(E1) | 0.1 | 8.2 | 69.3 | 93.8 | 101.1 | 99.8 | 102.1 | 101.5 |
| Example 2(E4) | 0.1 | 5.4 | 45.8 | 81.3 | 93.6 | 98.0 | 99.7 | 101.3 |
| Example 3(E10) | 0.1 | 0.2 | 11.2 | 71.1 | 93.3 | 98 | 100.2 | 99.8 |
| Example 4(E12) | 2.4 | 13.2 | 93.6 | 97.9 | 98.0 | 98.3 | 97.9 | 98.1 |
| Example 5(E13) | 2.8 | 3.2 | 60.8 | 95.6 | 98.1 | 97.5 | 98.2 | 98.3 |
| Example 6(E15) | 1.1 | 5.7 | 66.9 | 95.6 | 99.1 | 99.9 | 100.2 | 100.1 |
| Example 7(E22) | 2.1 | 11.3 | 72.5 | 92.6 | 96.8 | 97.8 | 98.2 | 98.6 |
| Comparative Example 1(D1) | 0.5 | 6.0 | 22.6 | 54.3 | 77.7 | 88.6 | 94.5 | 98.6 |
| Comparative Example 2(D2) | 5.4 | 27.5 | 94.4 | 95.2 | 95.2 | 95.6 | 94.8 | 94.9 |
| Comparative Example 3(D4) | 0.1 | 0.1 | 1.2 | 25.0 | 73.0 | 93.8 | 97.8 | 99.1 |
| Comparative Example 4(D5) | 0.1 | 0.2 | 0.5 | 9.2 | 39.5 | 78.5 | 88.6 | 96.8 |

The enteric-coated metformin hydrochloride preparation disclosed in WO2014107617A requires that less than 15%, 10% or 5% of the biguanide compound be released in a lag phase of two hours at acidic pH and at least ten minutes or fifteen minutes at pH 6.8, and at least 60% of the biguanide compound be released within 60 minutes at pH 6.8 after the lag phase, and at least 90% of the biguanide compound be released within 90 to 120 minutes at pH 6.8. It can be seen that the dissolution of D5 meets the requirements of the enteric-coated metformin hydrochloride preparation described in the said patent document WO2014107617A.

### Clinical trials and results

Treatment-naive patients who were intolerant to ordinary immediate-release metformin hydrochloride tablets (IR preparation) were randomly to four groups, administered metformin hydrochloride enteric-coated tablets of E1, D2, D5 and ordinary immediate-release metformin hydrochloride tablets (reference preparation, R, trade name Glucophage^{®}, commercially available) respectively, effects and adverse reactions were evaluated for metformin IR and DR with different release characteristics.

**Inclusion criteria:** Subjects who meet all of the following criteria be included in this trial:
(1) Age: 28 to 78 years old, male or female;
(2) Diagnosed with type 2 diabetes according to the WHO (1999) diabetes diagnostic criteria ;
(3) Patients who have not taken other hypoglycemic drugs and are intolerant to metformin IR, including intolerance to gastrointestinal reactions such as diarrhea, nausea, vomiting, bloating, indigestion and abdominal discomfort, and adverse reactions of the nervous system such as dizziness and headache, and patients who have various adverse reactions that may be related to metformin IR, patients should have been taking the drug for less than 2 weeks and are willing to take an alternative drug.
(4) Glycated hemoglobin (HbA_{1c}) ≥7.0% and ≤11.0% at the screening visit;
(5) Fasting blood glucose (FPG) ≤15mmol/L (270mg/dL) at the screening visit ;
(6) Body mass index (BMI) ≥19 kg/m ² and ≤35 kg/m ²;

**Dosage:** Replace metformin IR with the same dose of E1, D2, D5 and Glucophage. After replacement, increase the dose by 500 mg per week (take once in the morning and once in evening) until reaches 2000 mg/day. For those who have already reached 2000 mg/day, do not increase the dose and continue to take the replacement drug according to the original dose and method. For those who are still intolerant after changing the drug, the dose can be reduced to 1500 mg/day or 1000 mg/day.

During the treatment period, subjects who have other diseases that affects the evaluation of efficacy and adverse events may withdraw from the trial. For subjects who withdraw due to adverse events, the adverse events will be recorded and the subjects would be included in statistical analysis for safety review.

**Primary efficacy indicator:** Change in HbA_{1c} compared with baseline after 12 weeks of treatment.

### Secondary efficacy indicators:

(1) Changes in fasting blood glucose (FPG) compared with baseline at 4, 8, and 12 weeks of medication.
(2) Changes in body weight compared with baseline at 4, 8, and 12 weeks of medication.
(3) Changes in blood lipids (TC, TG, LDL-C, HDL-C) compared with baseline at 4, 8, and 12 weeks of medication.

**Safety evaluation:** adverse events were recorded during the study. Special attention was paid to the common gastrointestinal adverse reactions of metformin (including abdominal pain, diarrhea, constipation, abdominal distension, indigestion, etc.)

All medications (including combined medications) and adverse events of the patients were recorded in diary cards. The diary cards were collected at the next visit and medication compliance was calculated (by calculating the number of tablets used, generally requiring the actual number of tablets used to be > 80% of the theoretical amount). Patients with poor compliance were supervised. Subjects who took other hypoglycemic drugs were dropped from the study.

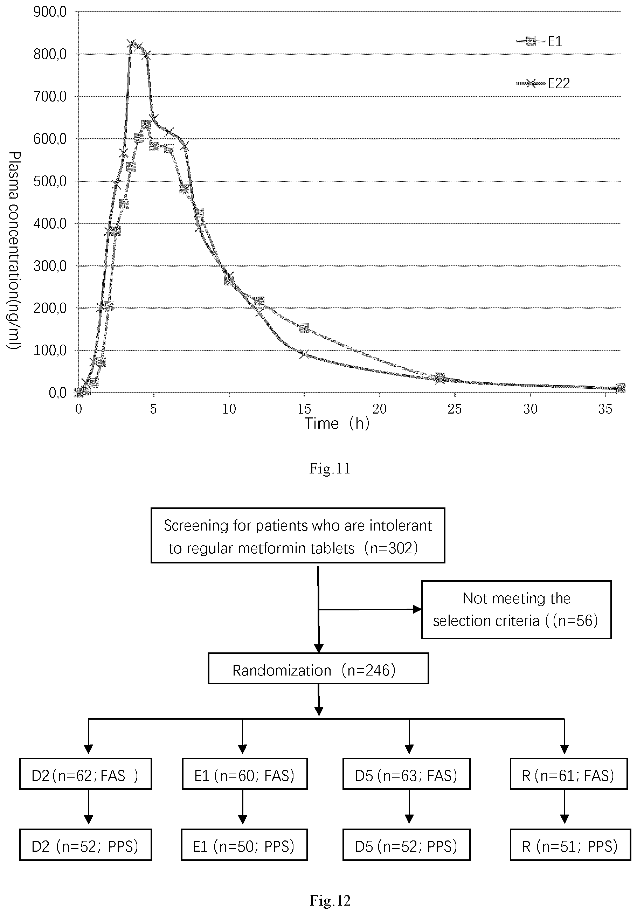

The trial process and subject distribution are shown in Table 4 and Figure 12. In Figure 12, FAS is the full analysis data set, which is all subjects who have taken the trial drug, and is used for safety / adverse event analysis. PPS is the per-protocol data set, which is all subjects who took the trial drug according to the protocol and obtained the 12 weeks' HbA1c, and it is used for efficacy analysis.

**Table 4: Trial process**

| **Stage** | **Screening period** | **Baseline period** | **Treatment period** | | |
|---|---|---|---|---|---|
| **Visit** | **Visit 1** | **Visit 2** | **Visit 3** | **Visit 4** | **Visit 5** |
| **Number of days** | **-D 14 -D0** | **D0** | **D28± 5** | **D56± 5** | **D84± 5** |
| Medical history / demographic data | X | | | | |
| Entry criteria | X | X | | | |
| Randomization | | X | | | |
| Distribute / collect medication and diary cards | | X | X | X | X |
| Adverse event records | | X | X | X | X |
| Treatment recommendations and medication instructions | X | X | X | X | X |
| Physical examination | X | X | X | X | X |
| Blood examination | X | X | X | X | X |
| Liver and kidney function examination | X | X * | X | X | X |
| HbA1c | X * | | | | X |
| FPG | X | X | X | X | X |
| Body weight | X | X | X | X | X |
| Blood lipids | X | X * | X | X | X |
| Uric acid | X | X * | X | X | X |

| | | | | | |
|---|---|---|---|---|---|
| **: No need to do this if done within 2 weeks; efficacy statistics are based on the value of the last examination before taking the medicine.* | | | | | |

### Results and discussion

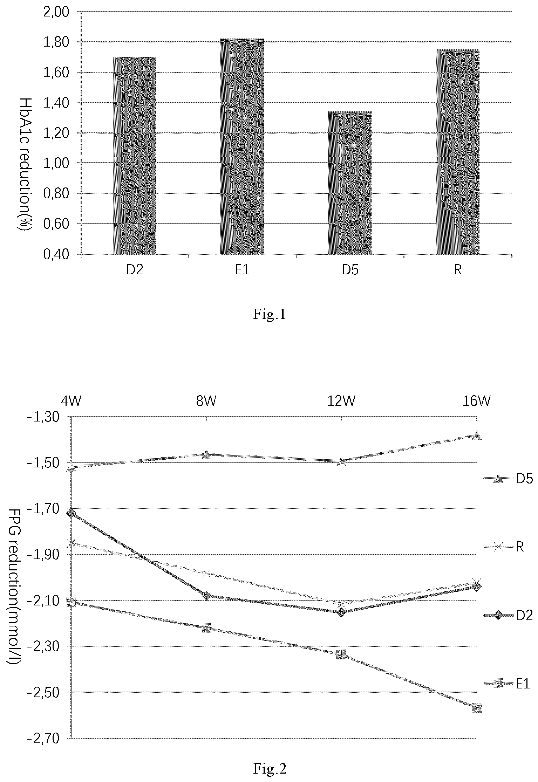

E1 had the best efficacy in terms of HbA1c, and fasting blood glucose also showed a similar trend. In addition, the reduction in fasting blood glucose showed an increasing trend as the duration of medication increased, indicating that E1 may have better long-term efficacy (see Figures 1-2 and Table 5. FPG at the 16^{th} week was an unscheduled test, with a small number of patients and a large time span, so no statistical analysis was performed). E1 was also superior to other preparations in terms of its effects on blood lipids, liver and kidney function. There was no significant difference between D2 and the control drug Glucophage in terms of hypoglycemic effect. D5 was significantly different from Glucophage (P<0.05, and the magnitude exceeded the clinical non-inferiority margin of 0.3-0.4).

**Table 5: Efficacy**

| | | D2 **(n=52)** | E1 **(n=50)** | D5 **(n=52)** | R **(n=51)** |
|---|---|---|---|---|---|
| HbA1c, (%) | | | | | |
| | Baseline | 8.24 (1.03) | 8.37 (0.91) | 8.44 (1.26) | 8.21 (1.17) |
| | End of treatment (3M) | 6.54 (0.58) | 6.55 (0.60) | 7.10 (1.05) | 6.45 (0.63) |
| | Reduction in 3M | 1.70 (0.93)** | 1.82 (1.08) ** | 1.34 (0.84) ** | 1.75 (1.19) ** |
| | p -value (vs R) | 0.82 | 0.88 | 0.04 | |

| FPG, mmol/l | | | | | |
|---|---|---|---|---|---|
| | Baseline | 9.19 (1.69) | 9.45 (1.75) | 9.21 (2.13) | 9.01 (2.27) |
| | Reduction in 1M | 1.72 (1.23) ** | 2.11 (1.70) ** | 1.52 (1.42) ** | 1.85 (1.94) ** |
| | Reduction in 2M | 2.08 (1.31)** | 2.22 (1.90) ** | 1.46 (1.34) ** | 1.98 (2.03) ** |
| | Reduction in 3M | 2.15 (1.27) ** | 2.34 (1.98) ** | 1.49 (1.44) ** | 2.11 (2.22) ** |
| | p -value (3M vs R) | 0.82 | 0.55 | 0.10 | |

| Weight, kg | | | | | |
|---|---|---|---|---|---|
| | Baseline | 71.7±11.8 | 72.3±12.6 | 73.2±12.1 | 72.6±11.1 |
| | Reduction in 1M | 0.46±0.30* | 0.58±0.35** | 0.46±0.34* | 0.56±0.36** |
| | Reduction in 2M | 0.64±0.45** | 0.71±0.46** | 0.52±0.40** | 0.67±0.48** |
| | Reduction in 3M | 0.81±0.55** | 0.88±0.56** | 0.63±0.50** | 0.82±0.52** |
| | p -value (3M vs R) | 0.91 | 0.75 | 0.37 | |

| | | | | | |
|---|---|---|---|---|---|
| ** : P<0.05 compared with baseline ; ** : P<0.01 compared with baseline* | | | | | |

**Table 6: Adverse Events**

| **Major adverse events** | D2 **(n=62)** | | | E1 **(n=60)** | | | D5 **(n=63)** | | | R **(n=61)** | | |
|---|---|---|---|---|---|---|---|---|---|---|---|---|
| | Screening | Trial | Ratio | Screening | Trial | Ratio | Screening | Trial | Ratio | Screening | Trial | Ratio |
| Nausea | 21 | 13 | 61.9% | 20 | 6 | 30.0%* | 26 | 8 | 30.8%* | 23 | 25 | 108.7% |
| Vomit | 6 | 3 | 50.0% | 8 | 3 | 37.5% | 11 | 3 | 27.3% | 8 | 6 | 75.0% |
| Stomach ache | 14 | 7 | 50.0% | 15 | 5 | 33.3% | 11 | 4 | 36.4% | 9 | 8 | 88.9% |
| Abdominal Discomfort | 5 | 4 | 80.0% | 8 | 3 | 37.5% | 10 | 3 | 30.0% | 11 | 8 | 72.7% |
| Abdominal Bloating | 18 | 10 | 55.6% | 13 | 4 | 30.8% | 17 | 6 | 35.3% | 17 | 15 | 88.2% |
| Acid reflux | 4 | 3 | 75.0% | 6 | 2 | 33.3% | 8 | 3 | 37.5% | 6 | 7 | 116.7% |
| Diarrhea | 31 | 27 | 87.1% | 32 | 23 | 71.9% | 35 | 45 | 128.6% | 26 | 28 | 107.7% |
| Constipation | 4 | 3 | 75.0% | 4 | 3 | 75.0% | 2 | 3 | 150.0% | 3 | 3 | 100.0% |
| Dizziness | 11 | 6 | 54.5% | 10 | 3 | 30.0% | 8 | 3 | 37.5% | 16 | 13 | 81.3% |
| Headache | 3 | 2 | 66.7% | 6 | 3 | 50.0% | 9 | 4 | 44.4% | 10 | 8 | 80.0% |
| Upper respiratory tract infection | 3 | 9 | 300.0% | 3 | 6 | 200.0% | 4 | 6 | 150.0% | 4 | 10 | 250.0% |
| **Main AE Total AE Lead to withdrawal** | 120 | 87 | 72.5% | 125 | 61 | 48.8%* | 141 | 88 | 62.4%* | 133 | 131 | 98.5% |
| | | 16 | 25.8% | | 7 | 11.7% | | 14 | 22.2% | | 21 | 34.4% |
| **Upper GI AE** | 68 | 40 | 58.8% | 70 | 23 | 32.9%* | 83 | 27 | 32.5%* | 74 | 69 | 93.2% |
| **Lower GI AE** | 35 | 30 | 85.7% | 36 | 26 | 72.2% | 37 | 48 | 129.7% | 29 | 31 | 106.9% |

| | | | | | | | | | | | | |
|---|---|---|---|---|---|---|---|---|---|---|---|---|
| *AE: adverse event* *Upper GI AEs (Upper gastrointestinal AEs) include: nausea, vomiting, abdominal pain, abdominal discomfort, bloating, acid reflux* *Lower GI AEs (Lower gastrointestinal AEs) include: diarrhea, constipation* *Ratio = adverse events during the trial period* / *adverse events during the screening period × 100% ;* ** : P<0.05 compared with R* | | | | | | | | | | | | |

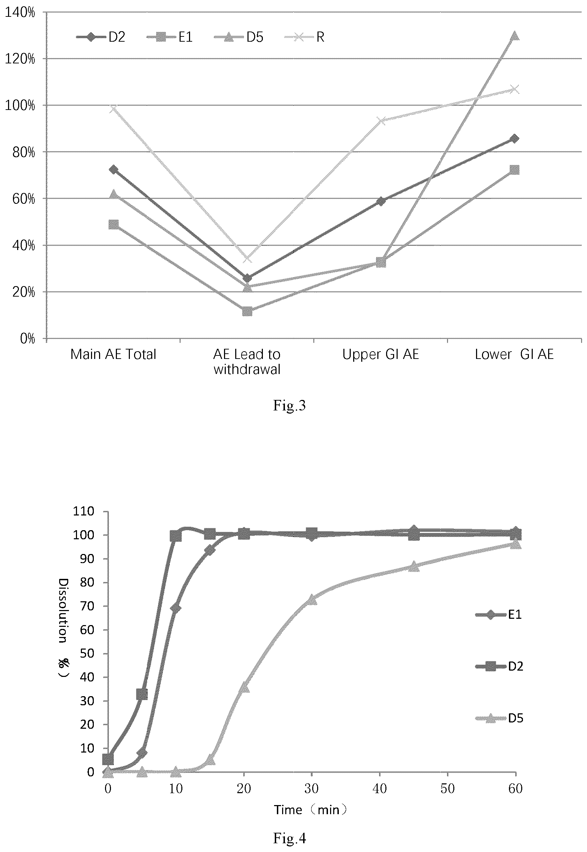

E1, D2, and D5 were significantly superior to Glucophage in terms of upper gastrointestinal adverse events (AEs), such as nausea, vomiting, abdominal pain, abdominal discomfort, abdominal distension, and acid reflux, especially E1 and D5. For patients who experienced upper gastrointestinal adverse events while taking metformin hydrochloride tablets, the probability of experiencing upper gastrointestinal AEs after switching to E1 and D5 was only 33% of that before the medication change, while it was 93% for Glucophage. The improvement of D2 was not very obvious, and the probability of upper gastrointestinal AEs after the change of medication was 59% of that before the change of medication. E1 also had certain advantages in other AEs (see Table 6 and Figure 3).

The inventors conducted further analysis on the patients in group D2 and found that the occurrence of upper gastrointestinal AE in this group was significantly related to the time of taking the medicine, patients who took the medicine before meals (30 minutes before meals) had less upper gastrointestinal AE than those who took the medicine after meals or during meals (E1 and D5 did not have such a phenomenon). This is obviously inconsistent with the adverse reaction characteristics of metformin reported in the literature. The literature shows that the adverse reactions of taking the medicine before meals are higher than those of taking it during meals and after meals. Therefore, the labeling of various countries require that metformin (ordinary preparations) be taken during meals (or immediately after meals) to reduce adverse gastrointestinal reactions.

After noticing the above phenomenon, the researchers compared adverse events of the drug before and after meals, the relationship between meal times and AEs shown in Table 7.

**Table 7 : Comparison of adverse events in patients in group D2 after administration of medication before and after meals**

| **Adverse Events** | Before meal | | | During and after meals | | | D2 group total (n=62) | | |
|---|---|---|---|---|---|---|---|---|---|
| | Screening | Trial | Ratio | Screening | Trial | Ratio | Screening | Trial | Ratio |
| Nausea | 9 | 3 | 33% | 9 | | 67% | 21 | 13 | 62% |
| Vomit | 2 | 0 | 0% | 3 | 6 2 | 67% | 6 | 3 | 50% |
| Stomach ache | 5 | 2 | 40% | 5 | 4 | 80% | 14 | 7 | 50% |
| Abdominal discomfort | 2 | 1 | 50% | 2 | 1 | 50% | 5 | 4 | 80% |
| Abdominal bloating | 6 | 2 | 33% | 8 | 5 | 63% | 18 | 10 | 56% |
| Acid reflux | 2 | 1 | 50% | 1 | 1 | 100% | 4 | 3 | 75% |
| Diarrhea | 11 | 7 | 64% | 9 | 6 | 67% | 31 | 27 | 87% |
| Dizziness | 4 | 2 | 50% | 5 | 3 | 60% | 11 | 6 | 55% |
| **Total upper GI AEs** | 26 | 9 | 35% | 28 | 19 | 68% | 68 | 40 | 59% |

| | | | | | | | | | |
|---|---|---|---|---|---|---|---|---|---|
| *upper GI(gastrointestinal) AE is the same as in the above table.* *Data that could not distinguish the correlation between medication* / *meal* /*AE were excluded because the diary card did not clearly record the time of medication and meal. Therefore, the number of people counted before meals + During meals and after meals was less than the total number of D2 groups.* | | | | | | | | | |

The inventors designed a dissolution test: simulated postprandial gastric juice (acetate buffer and milk were mixed in a ratio of 1:1, sodium hydroxide or hydrochloric acid was used to adjust the pH value to 5.0, acetate buffer contained 13.85g/L sodium chloride, 1.03g/L acetic acid, and 2.44g/L sodium acetate), paddle method, 75 rpm for D2 dissolution test, tablets began to break at 30 minutes, dissolution reached more than 75 % at 60 minutes, and metformin was completely dissolved at 90 minutes (detection method: high performance liquid chromatography, sample filtration and dilution (pre-column added if necessary), sulfonic acid cation exchange bonded silica gel as filler, 1.7% diammonium phosphate solution (phosphoric acid adjusted pH to 4.0) as mobile phase, detection wavelength of 233nm, column temperature of 30°C, flow rate of 1.3ml/min). The test results show that it is difficult to achieve the "enteric dissolution" effect when taking D2 after a meal.

The inventors re-performed the dissolution test of postprandial gastric juice with Example 4 (E12) and Example 6 (E15), and the test method was the same as above. The tablets of Example 4 (E12) began to break at about 60 minutes with a dissolution rate of about 20 %, and the dissolution rate reached more than 30 % at 90 minutes. The tablets of Example 6 (E15) began to swell and deform at about 60 minutes with a dissolution rate of about 10%, and the dissolution rate was about 25% at 90 minutes. Considering that the drug dissolved in the human stomach will be continuously diluted and discharged into the intestine, the concentration of metformin in the stomach should be much lower than that of the immediate-release tablets.

In order to better understand the dissolution of the product in the human body, especially considering that the influence of gastrointestinal peristalsis mechanical force is more obvious when taking the medicine after a meal, the inventor conducted a reciprocating barrel dissolution test, using simulated postprandial gastric juice (the composition of postprandial gastric juice and the detection method of metformin are the same as above) as the medium, and setting the reciprocating barrel reciprocating rate to 30dpm. As a result, D2 began to rupture at 25 minutes, and the dissolution rate reached more than 85% in 60 minutes. E12 began to rupture at about 50 minutes, the dissolution rate was about 25% at 60 minutes, and the dissolution rate was about 40% at 90 minutes. E15 had a dissolution rate of about 15% at 60 minutes and about 35% at 90 minutes. The experiment further shows that it is difficult to achieve the "enteric" effect when taking D2 after a meal.

When taking medicine during or after a meal, the drug often stays in the stomach longer than when taking medicine on an empty stomach, and the pH value is also higher, which can reach 4.5-5.0. According to the analysis of the pharmacokinetic and BA trial results, metformin enteric-coated tablets taken during meals can stay in the stomach for up to several hours, so the dissolution in pH4.5 medium is of great value to this product. The results of clinical and simulated postprandial gastric juice tests showed that when the dissolution in pH4.5 medium exceeded 20% (D2), the intragastric release increased significantly, and the adverse reactions also increased significantly, while when it was less than 15% (E12, E15), limited intragastric release could be achieved, and when it was less than 7.5 % (E4, E13, E22), it could achieve bioequivalence with the best target product (E1).

At present, the requirements of drug regulatory authorities in most countries for enteric-coated tablets are that the dissolution in a medium of pH 1.0 is less than 5 %~ 10% in 2 hours. D2 meets this requirement, which shows that this requirement may be difficult to ensure that the enteric effect is achieved when taking the medicine after meals. Since ordinary preparations of metformin hydrochloride (i.e., immediate release preparations) are taken after meals or during meals, many patients still habitually take metformin hydrochloride enteric-coated tablets after meals or during meals, so ensuring "enteric effect" after meals is more important for metformin hydrochloride. At the same time, products which can be taking before meals, after meals or during meals can significantly improve patients' compliance.

D5 were significantly improved in upper gastrointestinal reactions compared with those of Glucophage, but the lower gastrointestinal reactions were higher than those of Glucophage, D2 and E1. The incidence of lower gastrointestinal adverse events (mainly diarrhea) of D5 was 130% of that of ordinary tablets (IR), and if the duration of diarrhea was calculated, it was 156% of that of ordinary tablets. In addition, 5 patients in D5 had severe watery stools, 2 of which occurred after taking IR and worsened after switching to D5, and 3 have no diarrhea when taking IR and diarrhea appeared after switching to D5. The researchers judged that this symptom was an adverse reaction caused by the drug.

Since metformin is highly irritating to the gastrointestinal mucosa, the inventors consider that the watery stools is caused by drug irritating in the colorectal mucosa. According to the drug formulation and dissolution, D2 should be released in the stomach, duodenum and upper small intestine (depending on the time of drug administration), and E1 should be released in the small intestine. The small intestine has fast peristalsis and a large amount of intestinal fluid, so the drug is quickly diluted and has a weak irritation to the colon. D5 dissolves slowly and is likely to be released in the colon. The colon has slow peristalsis, less fluid, and less drug absorption, which may form high concentration locally and irritate the colon mucosa.

### Pharmacokinetic studies and results

In order to study the absorption of metformin enteric-coated preparations with different release characteristics, as well as their relationship with hypoglycemic effects and adverse reactions, the inventors conducted a pharmacokinetic study of the preparations.

Four products with different release characteristics in the above clinical trials were selected, namely, three metformin hydrochloride enteric-coated tablets E1, D2, and D5 (the dissolution curves are shown in Figure 4. Considering that the drug dissolved in acid will also be absorbed, D2 is the cumulative dissolution. Cumulative dissolution = dissolution + dissolution at 0 hour or dissolution in acid) and the reference preparation (metformin hydrochloride tablets, trade name: Glucophage^{®}) , and a pharmacokinetic study was conducted in healthy subjects, and the bioavailability of E1, D2, and D5 relative to Glucophage (R) was calculated.

### Method:

Inclusion criteria: subjects must meet all of the following criteria:
1) Healthy subjects: medical history, vital signs, physical examination, laboratory tests, electrocardiogram, chest X- ray and other related examinations are within the normal range, or the researchers judge that the abnormalities have no clinical significance.
2) Men and women aged 18 to 65 (including borderline values).
3) Male weight ≥50 kg, female weight ≥45 kg, body mass index between 19 and 28 (including the critical value) [ body mass index (BMI) = weight (kg) / height² (m²) ] ;

Before statistical analysis, if any of the following situations occurs, a comprehensive judgment will be made on whether to exclude the subject based on factors such as the period of the trial and the reason for withdrawal:
1) If the first sample is C ₘₐₓ and no early samples (within 30 minutes after administration) are collected, the pharmacokinetic data of the corresponding period of the subject will not be included in the evaluation;
2) If a subject vomits within twice the mediumn Tmax after administration, the pharmacokinetic data of the subject in the corresponding period will not be included in the evaluation.
3) If the plasma concentration before administration (0 hour concentration) is greater than 5% of the Cmax after administration, the pharmacokinetic data of the corresponding period of the subject will not be included in the evaluation.
4) Trial data may have large deviations or the PK parameters can not be calculated due to reasons such as violation of the dosing regimen, incorrect blood collection time, improper sample handling or storage, and poor subject compliance.

Dosage schedule: a four-period crossover design was used, with administration once on the first day of each period and a three-day washout period during the two-week period. Twenty subjects were randomly divided into four sequences at a ratio of 1:1:1:1. Subjects fasted for 10 h before administration. On the day of administration, the reference preparation (R: Glucophage, 1 tablet, 500 mg) or the test preparation (T: E1, D2, D5; all 500 mg) was taken fasting with 240 ml water. A standard lunch was eaten 4 h after taking the medicine.

The study design is shown in Table 8.

**Table 8: Study design**

| | **Period 1** | **Period 2** | **Period 3** | **Period 4** |
|---|---|---|---|---|
| Sequence 1 | R | D2 | E1 | D5 |
| Sequence 2 | D5 | R | D2 | E1 |
| Sequence 3 | E1 | D5 | R | D2 |
| Sequence 4 | D2 | E1 | D5 | R |

During the entire trial, the subjects ate standard meals provided by the research center, subjects avoided strenuous exercise and long-term lie in bed, and were prohibited from taking juice, tea, coffee, alcohol and other caffeinated or ethanol beverages, and smoking. If adverse events occurred during the trial, the researchers were required to follow up the subjects until the adverse events were alleviated or the symptoms disappeared.

Blood was collected at 0 h (within 60 min before drug administration) and 0.5 h, 1.0 h, 1.5 h, 2.0 h, 2.5 h, 3.0 h, 3.5 h, 4.0 h, 4.5 h, 5.0 h, 6.0 h, 7.0 h, 8.0 h, 10.0 h, 12.0 h, 15 h, and 24.0 h after drug administration in each period (a total of 18 blood collection points each period, a total of 72 blood collection points in 4 periods). 3 ml of peripheral venous blood was collected at each blood collection point and placed in a K2EDTA blood collection tube, centrifuged (1700 g, 2°C~8°C, set temperature at 4°C) for 10 min, and plasma was separated. The plasma samples were stored in a low-temperature refrigerator until detection. The drug concentration of metformin hydrochloride in plasma was determined by liquid chromatography - tandem mass spectrometry (HPLC-MS/MS) (Reference: Zhang D, Wang GC, Huang JQ, et al., LC-MS/MS determination of metformin in human plasma [ J ]. Chin J Pharm Anal (Journal of Drug Analysis), 2011, 31(2): 317-321; Zhang Dan, Post-marketing bioequivalence re-evaluation of metformin hydrochloride enteric-coated tablets in humans, Chinese Journal of Pharmaceutical Sciences, Vol. 47, No. 18, October 2012).

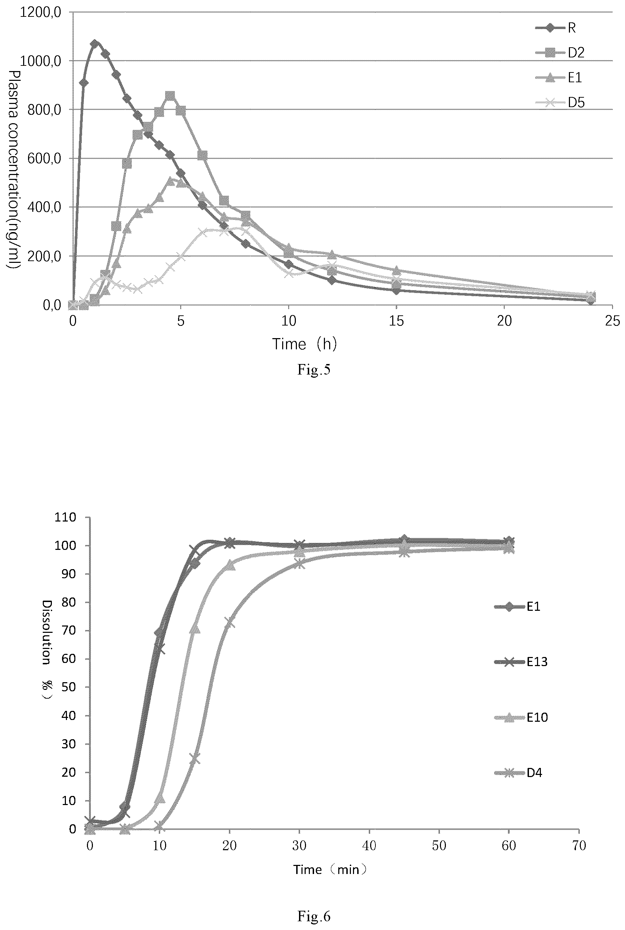

The results are as follows (Tables 9-10 and Figure 5).

**Table 9: Pharmacokinetic parameters (PKPS)**

| **Parameters (Mean±SD)** | **D2 (n=20)** | **E1 (n=18)** | **D5 (n=20)** | **R(n=19)** |
|---|---|---|---|---|
| C ₘₐₓ (ng/ml) | 982.99±254.50 | 710.51±299.89 | 567.26±399.85 | 1115.05±216.59 |
| AUC₀₋ₜ (h*ng/ml) | 5701.69±1235.03 | 4836.79±1636.07 | 3110.98±1614.21 | 6323.58±1333.26 |
| AUC_{0-∞}(h*ng/ml) | 5978.04±1217.67 | 5108.78±1716.44 | 3432.56±1614.64 | 6436.91±1375.07 |
| Tmax (h) | 4.50 (2.5-5.0 ) | 5.00 (3.0-12.0 ) | 7.00 (1.5-15.0 ) | 1.00 (0.5-2.0 ) |
| T _{1/2} (h) | 5.50±2.06 | 4.75±1.50 | 5.26±1.42 | 4.00±0.82 |

| | | | | |
|---|---|---|---|---|
| Note: Mean±SD represents the arithmetic mean ± standard deviation; Tmax is expressed as mediumn (minimum value -- maximum value). | | | | |

**Table 10: Relative bioavailability**

| | **Pharmacokinetic parameters** | **Geometric mean (T)** | **Geometric mean (R)** | **( T/R ) %** | **90% confidence interval** |
|---|---|---|---|---|---|
| D2/R | C ₘₐₓ (ng/ml) | 946.78 | 1111.90 | 85.15 | (71.01 - 102.11) |
| | AUC ₀₋ₜ (h*ng/ml) | 5540.72 | 6304.65 | 87.88 | (77.92 - 99.12) |
| | AUC _{0-∞} (h*ng/ml) | 5829.38 | 6417.82 | 90.83 | (81.31 - 101.47) |
| E1/R | C ₘₐₓ (ng/ml) | 623.26 | 1111.90 | 56.05 | (46.46 - 67.63) |
| | AUC ₀₋ₜ (h*ng/ml) | 4353.32 | 6304.65 | 69.05 | (60.96 - 78.21) |
| | AUC _{0-∞} (h*ng/ml) | 4606.11 | 6417.82 | 71.77 | (64.00 - 80.49) |
| D5/R | C ₘₐₓ (ng/ml) | 457.86 | 1111.90 | 41.18 | (34.34 - 49.38) |
| | AUC ₀₋ₜ (h*ng/ml) | 2750.27 | 6304.65 | 43.62 | (38.68 - 49.20) |
| | AUC _{0-∞} (h*ng/ml) | 3097.01 | 6417.82 | 48.26 | (43.11 - 54.02) |

The results showed that the dissolution behavior of metformin enteric-coated tablets was highly correlated with absorption. The relative bioavailability of the metformin hydrochloride enteric-coated tablets with the fastest (D2), medium (E1) and slowest (D5) release relative to Glucophage was 91%, 72%, and 48%, respectively. Dissolution was highly correlated with absorption (see Figures 4 and 5). Combined with clinical trials, dissolution was also significantly correlated with clinical efficacy and adverse reactions.

There was one case of nausea in the reference preparation group and one case of diarrhea in D5, both of which recovered on the next day. No adverse reactions were found in the other groups.

The enteric-coated preparations disclosed in WO2013103384A and WO2013103919A have a relative bioavailability reduced by 20% to 60%, preferably 40% to 60%, compared with immediate-release preparations having the same amount of the biguanide compound. It can be seen that D5 meets the enteric-coated preparations of metformin hydrochloride described in the aforementioned patent documents. Consistent with the results of a 16-week clinical study conducted by Elcelyx (Robert R. Henry et al.), D5 (bioavailability reduced by 52%) did not achieve the desired clinical efficacy. On the contrary, a relative bioavailability of 60 % to 80 % (E1) produced the best efficacy and the lowest adverse reactions.

### In vivo and in vitro correlation studies and results

The above clinical trials and pharmacokinetic studies of E1, D2 and D5 show that the efficacy, safety and absorption of metformin enteric-coated preparations are significantly correlated with drug dissolution. To further study the relationship between drug release characteristics and drug absorption, efficacy and safety, the inventors conducted the following in vitro and in vivo correlation studies.

### Trial 1:

To study the effect of drug release of the enteric-coated preparation on drug absorption, the following representative formulations are selected.
E1: used as a reference preparation (93.8% dissolution in pH 6.8 medium in 15 minutes and 101.1 % dissolution in 20 minutes).
E10: The tablet core is the same as E1. The dissolution in pH 6.8 medium is 71.1 % in 15 minutes and 93.3 % in 20 minutes.
E13: The tablet core is the same as E1. The dissolution in pH 6.8 medium is 95.6 % in 15 minutes and 98.1 % in 20 minutes.
D4: The tablet core is the same as E1. The dissolution rate in pH 6.8 medium is 25.0 % in 15 minutes and 73.0% in 20 minutes.
E1, E10, E13 and D4 in pH 6.8 medium are shown in Figure 6 (where E13 is the cumulative dissolution).

The study design is shown in Table 11.

**Table 11: Study design**

| **Trial 1** | Period 1 | Period **2** | Period 3 | Period 4 |
|---|---|---|---|---|
| Sequence 1 | E1 | E10 | E13 | D4 |
| Sequence 2 | D4 | E1 | E10 | E13 |
| Sequence 3 | E13 | D4 | E1 | E10 |
| Sequence 4 | E10 | E13 | D4 | E1 |

Because the previous pharmacokinetic trial showed that the plasma concentration was still high at 24 hours, this trial extended the blood collection time to 48 hours (adding two blood collection points at 36 hours and 48 hours), and other methods (inclusion criteria, dosing regimen, plasma concentration detection method, etc.) were the same as the pharmacokinetic test.

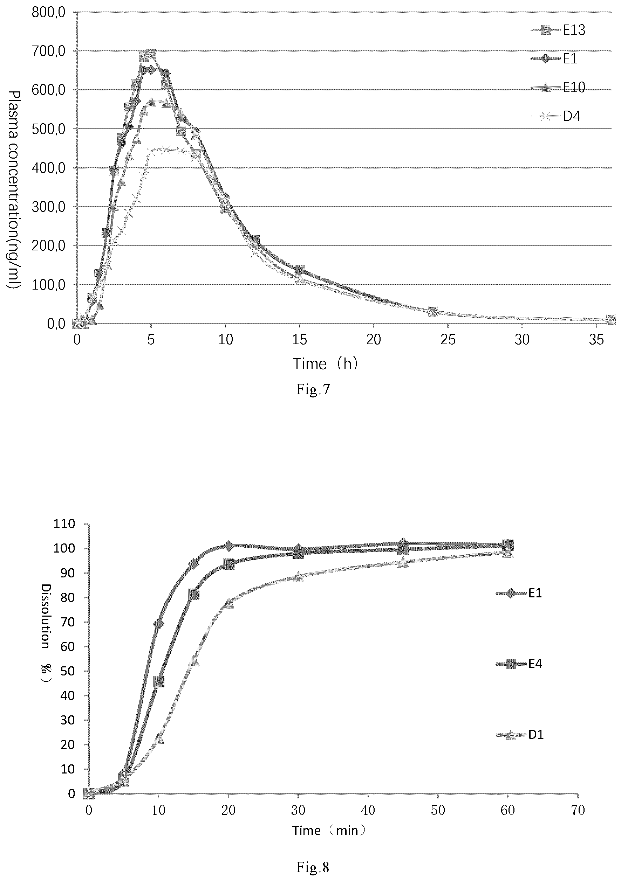

**Results:** see Tables 12, 13 and Figure 7.

**Table 12: Pharmacokinetic parameters (PKPS)**

| Parameters (Mean±SD ) | **E10 (n=20)** | **E13 (n=20)** | **D4 (n=19)** | **E1 (n=20)** |
|---|---|---|---|---|
| C ₘₐₓ (ng/ml) | 864.46±191.19 | 898.64±213.11 | 724.08±230.87 | 910.68±203.74 |
| AUC₀₋ₜ (h*ng/ml) | 5705.69±1459.71 | 6329.51±1271.74 | 4959.86±1256.55 | 6382.01±1233.42 |
| AUC_{0-∞} (h*ng/ml) | 5765.44±1458.68 | 6399.42±1288.50 | 5008.66±1259.49 | 6463.54±1259.21 |
| Tmax (h) | 5.00(2.50-10.00) | 4.50(2.50-12.00) | 6.00(1.50-10.00) | 5.00(1.50-8.00) |
| T _{1/2} (h) | 7.53±3.44 | 7.57±2.38 | 7.42±1.95 | 7.85±3.90 |

| | | | | |
|---|---|---|---|---|
| Note: Mean±SD represents the arithmetic mean ± standard deviation; Tₘₐₓ is expressed as mediumn (minimum value - maximum value). | | | | |

**Table 13: Relative bioavailability**

| | **Pharmacokinetic parameters** | **Geometric mean (T)** | **Geometric mean (E1)** | **( T/E1 ) %** | **90% confidence interval** |
|---|---|---|---|---|---|
| E13/E1 | C ₘₐₓ (ng/ml) | 872.10 | 886.86 | 98.34 | (90.14 - 107.28) |
| | AUC ₀₋ₜ (h*ng/ml) | 6209.89 | 6264.48 | 99.13 | (91.40 - 107.51) |
| | AUC _{0-∞} (h*ng/ml) | 6278.26 | 6342.85 | 98.98 | (91.25 - 107.37) |
| D4/E1 | C ₘₐₓ (ng/ml) | 694.99 | 886.86 | 78.36 | (71.72 - 85.62) |
| | AUC ₀₋ₜ (h*ng/ml) | 4823.05 | 6264.48 | 76.99 | (70.88 - 83.62) |
| | AUC _{0-∞} (h*ng/ml) | 4871.39 | 6342.85 | 76.80 | (70.70 - 83.43) |
| E10/E1 | C ₘₐₓ (ng/ml) | 846.18 | 886.86 | 95.41 | (87.46 - 104.09) |
| | AUC ₀₋ₜ (h*ng/ml) | 5544.65 | 6264.48 | 88.51 | (81.61 - 96.00) |
| | AUC _{0-∞} (h*ng/ml) | 5605.27 | 6342.85 | 88.37 | (81.47 - 95.86) |

E13 is bioequivalent to E1, with a BA (relative bioavailability) of about 99%. Although E10 is bioequivalent to E1, its BA is only 88% of that of E1. D4 has significantly reduced absorption, with a BA of only 77%, and is not bioequivalent to E1. The results further show that the in vitro dissolution (especially the dissolution in a pH 6.8 medium) of the metformin hydrochloride enteric-coated tablets of the present invention is well consistent with its in vivo absorption.

One adverse reaction (diarrhea) occurred on D4, and no adverse reactions were found in other groups.

### Trial 2:

To study the effect of drug release of the enteric-coated preparation on drug absorption, the following representative formulations are selected.
E1: used as a reference preparation (R). Dissolution is 93.8% in pH 6.8 medium within 15 minutes.
E4: Dissolution is 81.3 % in pH 6.8 medium in 15 minutes, and 93.6 % in 20 minutes.
D1: Dissolution is 54.3% in pH 6.8 medium in 15 minutes, and 77.7% in 20 minutes.

The dissolution curves in pH 6.8 medium of E1, E4 and D1 are shown in Figure 8.

The study design is shown in Table 14.

**Table 14: Study design**

| **Trial 2** | Period 1 | Period **2** | Period 3 |
|---|---|---|---|
| Sequence 1 | E1 | E4 | D1 |
| Sequence 2 | D1 | E1 | E4 |
| Sequence 3 | E4 | D1 | E1 |

The trial methods (inclusion and exclusion criteria, dosing regimen, plasma concentration detection method, etc.) are the same as those in Test 1.

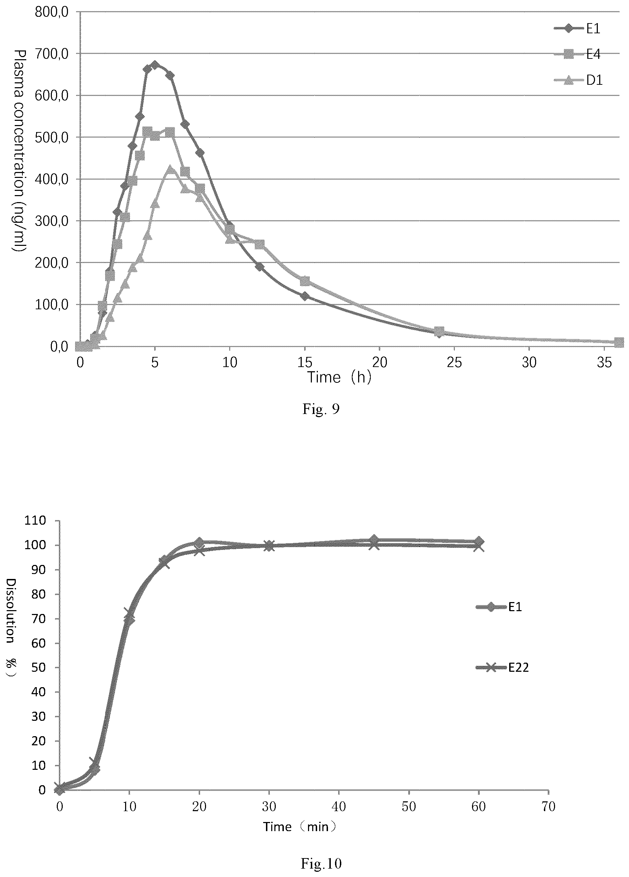

**Results:** see Tables 15, 16 and Figure 9.

**Table 15: Pharmacokinetic parameters (PKPS)**

| **Parameters (Mean±SD)** | **D1(n=18)** | **E4 (n=18)** | **E1 (n= 18 )** |
|---|---|---|---|
| Cₘₐₓ (ng/ml) | 718.52±145.13 | 850.39±248.99 | 882.52±231.72 |
| AUC ₀₋ₜ (h*ng/ml) | 4703.92±1076.55 | 5617.34±1590.36 | 5990.16±1652.69 |
| AUC _{0-∞} (h*ng/ml) | 4752.18±1083.80 | 5674.26±1606.71 | 6054.96±1667.50 |
| Tmax (h) | 6.00 ( 3.00-12.00 ) | 5.00 ( 2.00-12.00 ) | 4.50 ( 2.50-7.00 ) |
| T _{1/2} (h) | 8.17±2.47 | 9.06±2.39 | 8.25±2.53 |

| | | | |
|---|---|---|---|
| Note: Mean±SD represents the arithmetic mean ± standard deviation; T ₘₐₓ is expressed as mediumn (minimum value - maximum value). | | | |

**Table 16: Relative bioavailability**

| | **Pharmacokinetic parameters** | **Geometric mean (T)** | **Geometric mean (E1)** | **(T/E1) %** | **90% confidence interval** |
|---|---|---|---|---|---|
| D1/E1 | C ₘₐₓ (ng/ml) | 704.12 | 848.44 | 82.99 | (74.01 - 93.06) |
| | AUC ₀₋ₜ (h*ng/ml) | 4580.00 | 5753.38 | 79.61 | (73.50 - 86.22) |
| | AUC _{0-∞} (h*ng/ml) | 4627.87 | 5816.59 | 79.56 | (73.41 - 86.24) |
| E4/E1 | C ₘₐₓ (ng/ml) | 816.50 | 848.44 | 96.24 | (85.83 - 107.91) |
| | AUC ₀₋ₜ (h*ng/ml) | 5394.94 | 5753.38 | 93.77 | (86.58 - 101.56) |
| | AUC _{0-∞} (h*ng/ml) | 5449.70 | 5816.59 | 93.69 | (86.44 - 101.55) |

E4 is bioequivalent to E1, but its BA is only 94% of that of E1. D1 is not bioequivalent to E1, and its BA is only about 80% of that of E1. The trial results show that the disintegration time of the tablet core has a significant effect on the dissolution and absorption of the drug.

No adverse reactions were observed in any group.

### Trial 3:

To study the equivalence of capsules with similar release conditions with E1, the following representative formulations are selected.
E1: Used as a reference preparation (R). Dissolution is 93.8% in pH 6.8 medium within 15 minutes.
E22: Dissolution is 92.6% in pH 6.8 medium in 15 minutes, and 97.8% in 20 minutes.

The dissolution curves of E1 and E22 in pH 6.8 medium are shown in Figure 10.

The study design is shown in Table 17.

**Table 17: Study design**

| **Trial 3** | Period 1 | Period **2** |
|---|---|---|
| Sequence 1 | E1 | E22 |
| Sequence 2 | E22 | E1 |

The trial methods (inclusion and exclusion criteria, dosing regimen, plasma concentration detection method, etc.) are the same as those in Trial 1.

**Results:** see Tables 18, 19 and Figure 11.

**Table 18: Pharmacokinetic parameters (PKPS)**

| **Parameters (Mean±SD)** | **E22(n=20)** | **E1(n=20)** |
|---|---|---|
| Cₘₐₓ(ng/mL) | 865.04±215.99 | 890.04±319.58 |
| AUC₀₋ₜ(h*ng/mL) | 6374.63±1745.38 | 6089.29±2337.33 |
| AUC_{0-∞}(h*ng/mL) | 6416.88±1758.57 | 6143.82±2357.05 |
| Tₘₐₓ(h) | 3.5 (2.5-4.5) | 4.5 (2.5-8) |
| T_{1/2}(h) | 6.60±1.26 | 6.94±2.84 |

| | | |
|---|---|---|
| Note: Mean±SD represents the arithmetic mean ± standard deviation; T ₘₐₓ is expressed as mediumn (minimum value - maximum value). | | |

**Table 19: Relative bioavailability**

| **Pharmacokinetic parameters** | **Geometric mean (T)** | **Geometric mean (E1)** | **(T/E1 ) %** | **90% confidence interval** |
|---|---|---|---|---|
| Cₘₐₓ(ng/ml) | 836.33 | 830.66 | 100.68 | (89.84 - 112.84) |
| AUC₀₋ₜ(h*ng/ml) | 6102.31 | 5656.78 | 107.88 | (98.69 - 117.92) |
| AUC_{0-∞}(h*ng/ml) | 6142.24 | 5707.94 | 107.61 | (98.37 - 117.71) |

E22 and E1 are bioequivalent. The trial results show that the capsules with similar in vitro dissolution (especially the dissolution in pH 6.8 medium) have good consistency in absorption with the tablets. No adverse reactions were observed for E22 and E1.

The absorption site of metformin hydrochloride is the upper part of the small intestine. It is generally believed that the dissolution in pH 6.0 medium better reflects the drug release in the upper part of the small intestine, and its dissolution is an important factor affecting the absorption and efficacy of this type of drug. However, the present invention surprisingly found that the absorption of this product is mainly related to the dissolution in pH 6.8 medium. For a particular drug, BA basically depends on the formulation and process of the product. The pharmaceutical industry often uses dissolution characteristics to distinguish the differences in formulation and processes and hopes to predict in vivo absorption or bioavailability (BA) based on dissolution behavior. Compared with BA, the test of dissolution is much simpler and faster. If the correlation between dissolution and BA can be established, it will be of great value for selecting appropriate formulation and processes and predicting drugability. The above clinical trials, pharmacokinetic studies (E1, D2, D5) and in vitro and in vivo correlation studies (E1, E10, E13, D4; E1, E4, D1; E1, E22) all show that the enteric-coated metformin hydrochloride preparations with limited release in pH 4.5 medium (less than 7.5-15% release in 120 minutes) and rapid dissolution in pH 6.8 medium (more than 85% dissolution in 20 minutes, especially more than 85% dissolution in 15 minutes) can achieve the best therapeutic effect and the lowest adverse reactions. Their bioavailability is about 60-80% relative to metformin hydrochloride IR preparations (such as Glucophage).

## Claims

1. An enteric-coated preparation of a biguanide compound, which is an oral dosage form made of a therapeutically effective amount of a biguanide compound and a pharmaceutically acceptable excipient, **characterized in that**: the dissolution rate of the biguanide compound in the enteric-coated preparation in a pH 4.5 medium within 120 minutes is not higher than 15%, and the dissolution rate of the biguanide compound in a pH 6.8 medium within 20 minutes is not lower than 85%.

2. The enteric-coated preparation according to claim 1, **characterized in that** the dissolution rate of the biguanide compound in the enteric-coated preparation in a pH 4.5 medium within 120 minutes is not higher than 7.5%, and the dissolution rate of the biguanide compound in a pH 6.8 medium within 15 minutes is not lower than 85%.

3. The enteric-coated preparation according to claims 1-2, **characterized in that**, compared with an immediate-release preparation containing an equal amount of the biguanide compound, the said enteric-coated preparation has a relative bioavailability of 60% to 80%.

4. The enteric-coated preparation according to claims 1-3, wherein the biguanide compound is metformin or a pharmaceutically acceptable salt thereof; preferably, the biguanide compound is metformin hydrochloride.

5. The enteric-coated preparation according to claims 1-4, wherein the oral dosage form is a tablet, capsule or granule.

6. Use of a biguanide compound or a salt thereof for preparing a medicament for reducing adverse events caused by a biguanide compound, wherein the medicament is an enteric-coated preparation according to claims 1-5.

7. Use of a biguanide compound or a salt thereof in the preparation of a medicament for treating metabolic disorders, wherein the medicament is an enteric-coated preparation according to claims 1-5.

8. Use of a biguanide compound or a salt thereof in the preparation of a medicament for diabetes, wherein the medicament is an enteric-coated preparation according to claims 1-5.

9. Use of a biguanide compound or a salt thereof in the preparation of a medicament for reducing body weight, wherein the medicament is an enteric-coated preparation according to claims 1-5.

10. Use of a biguanide compound or a salt thereof for preparing a drug that can be taken before, after or during meals to improve patients' compliance with medication, wherein the drug is an enteric-coated preparation according to claims 1 to 5.
